Europäisches Patentamt

European Patent Office

Office européen des brevets

(19)

(11) Veröffentlichungsnummer : **0 009 076**
**B1**

(12) **EUROPÄISCHE PATENTSCHRIFT**

(45) Veröffentlichungstag der Patentschrift :
29.07.81

(51) Int. Cl.³ : **C 12 Q   1/48**

(21) Anmeldenummer : 79102247.8

(22) Anmeldetag : 03.07.79

(54) Verfahren zur quantitativen enzymatischen Bestimmung von ADP und Reagenz zur Durchführung des Verfahrens.

(30) Priorität : 08.08.78 DE 2834704

(43) Veröffentlichungstag der Anmeldung :
02.04.80 (Patentblatt 80/07)

(45) Bekanntmachung des Hinweises auf die Patenterteilung : 29.07.81 Patentblatt 81/30

(84) Benannte Vertragsstaaten :
BE CH DE FR GB IT LU NL SE

(56) Entgegenhaltungen :
DE - A - 2 061 984
DE - A1 - 2 518 862
DE - B1 - 2 412 354
US - A - 4 042 462

(73) Patentinhaber : **BOEHRINGER MANNHEIM GMBH**
**Sandhofer Strasse 112-132 Postfach 31 01 20**
**D-6800 Mannheim 31-Waldhof (DE)**

(72) Erfinder : **Deneke, Ulfert, Dr.**
**Ammerhöfe 80 1/2**
**D-8123 Peissenberg (DE)**
Erfinder : **Michal, Gerhard, Dr.**
**Kreuzeckstrasse 19**
**D-8132 Tutzing (DE)**
Erfinder : **Beutler, Hans-Otto, Dr.**
**Zugspitzstrasse 28**
**D-8132 Tutzing (DE)**

(74) Vertreter : **Weickmann, Heinrich, Dipl.-Ing Patentan-**
**wälte Dipl.Ing.H.Weickmann et al**
**Dipl.Phys.Dr.K.Fincke Dipl.Ing.F.A.Weickmann**
**Dipl.Chem.B.Huber, Dr.-Ing.H.Liska Möhlstrasse 22**
**D-8000 München 86 (DE)**

# 0 009 076

Verfahren zur quantitativen enzymatischen Bestimmung von ADP und Reagenz zur Durchführung des Verfahrens

Die Erfindung betrifft ein Verfahren zur quantitativen enzymatischen Bestimmung von ADP in wässriger Lösung.

Schon seit langem besteht in der Biochemie an der Messung des ADP und der Messung ADP-liefernder Reaktionen grosses Interesse. ADP-Bildung ist das Maß für den ATP-Umsatz, also die Verwertung des zentralen Energieträgers des Organismus. Aber auch in der klinischen Chemie hat die ADP-Messung große Bedeutung, wenn Herz-, Leber- und Nierenerkrankungen diagnostiziert werden. Der ADP-Nachweis im Zuge ADP-liefernder Reaktionen hat ebenfalls in der Biochemie und klinischen Chemie große Bedeutung. Hier sei der Nachweis der Pyruvat-kinase und der Creatin-kinase für Enzymbestimmungen und der Creatinin- und Glycerinnachweis für Metaboliten-Bestimmungen genannt.

Die Bestimmung von ADP in biologischen Flüssigkeiten, wie Seren, Gewebe, bakteriell kontaminiertem wässrigem Material oder anderen wässrigen Flüssigkeiten kommt daher große praktische Bedeutung zu.

Für den enzymatischen Nachweis von ADP wurden drei verschiedene Verfahren angewendet. Das erste beruht auf der Phosphorylierung des ADP zum ATP mit PEP (Phosphoenolpyruvat) und PK (Pyruvat-kinase) und Nachweis des entstehenden Pyruvats mit NADH/LDH (Lactatdehydrogenase).

A. 1. ADP + PEP $\xrightarrow{\text{PK}}$ ATP + Pyruvat

2. Pyruvat + NADH + H$^+$ $\xrightarrow{\text{LDH}}$ Lactat + NAD$^+$

Dieses Verfahren ist schon lange bekannt und beschrieben in Nature *178,* 632 (1956). Es bildet auch einen Teil des Verfahrens gemäß DE-A-2 412 354.

Die beiden anderen Verfahren beruhen ebenfalls auf der Phosphorylierung des ADP, z.B. mit Creatinphosphat und CK und Nachweis des gebildeten ATP

B. 1. Phosphoglycerinaldehyd + NAD + Phosphat $\xrightarrow{\text{GADPH}}$ 1,3-Diphosphoglycerat + NADH + H$^+$

2. 1,3-Diphosphoglycerat + ADP $\xrightarrow{\text{PGK}}$ 3-Phosphoglycerat + ATP

PGK = Phosphoglycerinkinase

GAP – DH = Glycerinaldehydphosphatdehydrogenase

Auch diese Reaktion ist lange bekannt und beschrieben in BBA *1,* 292 (1947).

C. 1. ADP + Creatin-phosphat $\xrightarrow{\text{CK}}$ ATP + Creatin

2. ATP + Glucose $\xrightarrow{\text{HK}}$ G-6-P + ADP

3. Glucose-6-phosphat + NAD$^+$ $\xrightarrow{\text{G-6-PDH}}$ Gluconat-6-phosphat + NADH + H$^+$

HK = Hexokinase

G-6-PDH = Glucose-6-phosphatdehydrogenase

Diese Reaktion wird bereits seit mindestens 1955 verwendet, beschrieben wird sie z.B. in A. Kornberg, Methods of Enzym. *2,* 497 (1955). Die parallele Reaktion mit NADP ist in der DE-A-2 518 862 beschrieben.

Diese bekannten Methoden weisen nun verschiedene Mängel auf, die ihre Anwendung erschweren oder verteuern. Zwar ist der Nachweis nach A grundsätzlich geeignet, als Indikatorsystem für Reaktionen zu dienen, die aus ATP ADP liefern und hat sich zu diesem Zweck durchgesetzt, es wird aber die Abnahme der NADH-Extinktion gemessen. In solchen Tests kann aus meßtechnischen Gründen nur eine begrenzte Anfangsextinktion an NADH vorhanden sein. Das hat zur Folge, daß das hier verwendete Indikatorenzym LDH nicht mit seinem Substrat NADH gesättigt ist und demnach nicht seine maximale Reaktionsbeschwindigkeit erreicht. Das muß durch erhöhten Enzymeinsatz ausgeglichen werden. Dies ist nicht nur unwirtschaftlich, sondern beinhaltet auch die Gefahr, daß andere störende Enzymaktivitäten in größerem Maße in das Testsystem eingeschleppt werden. Ein weiterer Nachteil ergibt sich daraus, daß bei hohen ADP-Konzentrationen das NADH schon verbraucht sein kann, bevor vollständiger Umsatz eingetreten ist. Man mißt dann falsch zu niedrige Werte.

Ein gravierender Mangel des Verfahrens B beruht darin, daß nach dem Prinzip der vorgeschalteten Indikatorreaktion eine bestimmte Menge ADP zwar eine stöchiometrisch errechenbare NADH-Bildung verursacht, jedoch daß keine ganzzahlige Stöchiometrie vorliegt und daher die Berechnung sehr kompliziert wird. Eine Anwesenheit von ATP verursacht außerdem eine zusätzliche Verschiebung des Gleichgewichts, was die Rechnung weiter erschwert. Daher wird dieses Verfahren in der Praxis kaum angewendet. Außerdem ist eine Verstärkung des Meßsignals durch nachgeschaltete Formazanreaktion nicht möglich.

Diese Nachteile besitzt das Verfahren C nicht. Dafür kommen zwei andere Mängel zum Tragen. Da das Verfahren C darauf beruht, ADP zu ATP zu phosphorylieren und dieses ATP dann nachzuweisen, ist es einleuchtend, daß dieses Verfahren nicht dazu geeignet ist, aus ATP im gekoppelten Test gebildetes ADP nachzuweisen. Wie im weiteren ausgeführt, besteht aber großes Interesse daran, viele Reaktionen zu messen, die aus ATP ADP liefern. Es ist aber anzustreben, für diese Nachweise stets dasselbe Indikatorsystem zu verwenden, weil die benötigten Enzyme und Reagentien um so günstiger produziert werden können, je größer die Produktionsansätze gemacht werden. Zum anderen ist es grundsätzlich nachteilig, Messungen im UV-Licht durchzuführen, da Meßeinrichtungen zur Messung im UV-Licht stets wesentlich teurer und aufwendiger sind als Meßeinrichtungen für die Messung im sichtbaren Licht.

Der Erfindung liegt daher die Aufgabe zugrunde, ein Verfahren zur Bestimmung von ADP zu

2

schaffen, welches die Nachteile der bekannten Verfahren nicht aufweist. Insbesondere ist es ein Ziel der Erfindung, ein derartiges Verfahren zu schaffen, welches kurze Meßzeiten und nur eine Inkubation erfordert, die Absorptionszunahme oder bei Enzymbestimmungen die Absorptionszunahme pro Minute mißt, eine Messung im sichtbaren Licht gestattet, damit einfachere photometrische Einrichtungen benutzt werden können und gleichzeitig an verschiedene Reaktionen gekoppelt werden kann, welche ADP aus ATP liefern.

Gelöst wird diese Aufgabe erfindungsgemäß durch ein Verfahren zur quantitativen enzymatischen Bestimmung von Adenosindiphosphat (ADP) in wässriger Lösung durch Phosphorylierung mit einer Kinase in Gegenwart eines phosphorylierten Substrats derselben unter Bildung von ATP und entphosphoryliertem Substrat, welches dadurch gekennzeichnet ist, daß das entphosphorylierte Substrat, gegebenenfalls unter Zwischenschaltung einer weiteren Hilfsreaktion mit NAD(P) in Gegenwart einer Dehyrogenase umgesetzt und gebildetes NAD(P)H direkt gemessen oder mit einem Tetrazoliumsalz in Gegenwart eines Elektronenüberträgers umgesetzt wird unter Bildung eines Formazans und letzteres gemessen wird.

Das Grundprinzip des erfindungsgemäßen Verfahrens besteht also darin, nicht wie bisher, mit einer Reaktion zu koppeln, in der NADH verbraucht wird, sondern umgekehrt, mit einer Reaktion, in welcher NAD oder NADP reduziert werden zum NADH bzw. NADPH. Für das Verfahren der Erfindung sind mehrere Ausführungsformen möglich, da sich einige verschiedene derartige Reaktionen als mit quantitativen Reaktionsabläufen koppelbar herausgestellt haben.

Es ist jedoch nicht in jedem Falle möglich, das entphosphorylierte Substrat der ersten Reaktion, bei welcher ADP aufphosphoryliert wird zu ATP direkt mit dem NAD oder NADP und einer Dehydrogenase umzusetzen. In solchen Fällen muß eine Hilfsreaktion zwischengeschaltet werden, insbesondere eine Decarboxylierungsreaktion, Transaminierungsreaktion oder Kupplung mit einer Hexose oder Pentose.

Desgleichen ist es gelegentlich günstig, gebildetes NAD(P) nicht direkt zu messen, sondern in einen Farbstoff zu überführen, indem ein geeignetes Tetrazoliumsalz in Gegenwart eines Elektronenüberträgers, wie Diaphorase, zum entsprechenden gefärbten Formazan umgesetzt wird und die gebildete Färbung gemessen wird. Geeignete Tetrazoliumsalze und Elektronenüberträger sind dem Fachmann bekannt. Lediglich beispielsweise seien hier 3-(4,5-Dimethylthiazolyl-2)-2,5-diphenyltetrazoliumbromid, Nitroblau, Tetrazoliumchlorid und 2-(p-Jodphenyl)-3-(p-nitrophenyl)-5-phenyl-tetrazoliumchlorid erwähnt.

Gemäß einer bevorzugten Ausführungsform des erfindungsgemäßen Verfahrens wird dieses mit Galactose-kinase und Galactose-dehydrogenase in der durch die nachstehenden Gleichungen veranschaulichten Reaktionsfolge durchgeführt :

D. 1. Gal-1-P + ADP $\xrightarrow{\text{Gal-kinase}}$ Galactose + ATP

2. Galactose + NAD$^+$ $\xrightarrow{\text{Gal-DH}}$ Galactonat + NADH + H$^+$

Gal-1-P = Galactose-1-phosphat

Gal-kinase = Galactose-kinase

Gal-DH = Galactose-dehydrogenase

Diese Gleichungen sind an sich seit langem bekannt aus Biochem. J. 44, 460 (1949) und J. Biol. Chem. 227, 745 (1957).

Überraschenderweise wurde jedoch nunmehr gefunden, daß es möglich ist, diese Reaktionen miteinander und mit Reaktionen zu koppeln, die aus ATP ADP bilden. Hierdurch wird die Absorptionszunahme durch das gebildete NADH zum Maß der Menge an ADP oder, falls bei den gekoppelten Reaktionen, die ADP aus ATP bilden, Enzymaktivitäten gemessen werden, die Absorptionszunahme pro Zeiteinheit durch das gebildete NADH zum Maß für die gesuchte Enzymaktivität. Eine derartige Kopplung wurde bisher nicht für möglich gehalten, wie schon allein daraus hervorgeht, daß seit mehr als 20 Jahren nach einem befriedigenden Verfahren zur Bestimmung von ADP gesucht wird und auch die Reaktionen schon so lange bekannt sind.

Gemäß einer besonders vorteilhaften Ausführungsform dieser Ausführungsform wird noch eine Reaktion nachgeschaltet, welche mit einem Tetrazoliumsalz in Gegenwart eines Elektronenüberträgers zur Rückoxidation des gebildeten NADH unter gleichzeitiger Bildung von gefärbtem Formazan führt, welches leicht im sichtbaren Licht gemessen werden kann. In einer bevorzugten Ausführungsform entspricht dies der nachstehenden Gleichung :

1. NADH + MTT$^+$ $\xrightarrow{\text{Diaphorase}}$ Formazan + NAD$^+$

MTT = 3-(4,5-Dimethylthiazolyl-2)-2,5-diphenyl-tetrazoliumbromid.

Als Elektronenüberträger wird die aufgeführte Diaphorase bevorzugt. Es können jedoch auch andere Elektronenüberträger, die dem Fachmann bekannt sind, verwendet werden. Als sehr gut geeignet hat sich dabei insbesondere Phenazinmethosulfat (PMS) und Phenanthrolinmethosulfonat erwiesen.

Als Tetrazoliumsalz wird das bereits erwähnte MTT bevorzugt. Es können aber auch andere Tetrazoliumsalze verwendet werden. Gute Ergebnisse wurden insbesondere auch mit Nitroblau-tetrazoliumchlorid (NBT) und 2-(p-Jodo-phenyl)-3-(p-nitrophenyl)-5-phenyl-tetrazoliumchlorid (INT) erzielt. Die Auswahl des Tetrazoliumsalzes erfolgt unter Berücksichtigung der Löslichkeit des gebildeten Formazanfarbstoffs. Weist letzterer nämlich eine geringe Löslichkeit auf, so können bei hohen ADP-Konzentrationen Ausfällungen des Farbstoffs erfolgen, welche zu Meßschwierigkeiten führen. Aus diesem Grunde wird zweckmäßig bei Durchführung des erfindungsgemäßen Verfahrens unter Messung eines Forma-

3

zanfarbstoffs ein oberflächenaktives Mittel zugesetzt, welches das Löslichkeitsverhalten des Farbstoffs verbessert. Beispiele für geeignete oberflächenaktive Mittel sind Desoxycholsäure, Saponin, Alkylarylpolyäthylenglykol-ester und -äther, Sorbitanester, wie Sorbimacrogololeat, Polyäthylenglykollauryläther und dergleichen. Die Konzentration an oberflächenaktivem Mittel liegt im allgemeinen zwischen 0,3 und 3 % im Test.

Zulässige Schwankungsbreite :

Die Art des Puffers ist unkritisch. Geeignete sind z.B. Tris-, Tra-, Imidazol-, Succinat, Glycin-, Glycyclglycin-puffer.

Pufferstärke 0,005 bis 0,5 Mol/l

pH 6 bis 10,5

Gal-Kinase 0,1 bis 20 U/ml

Gal-DH 0,05 bis 20 U/ml.

Auch die anderen, unten näher beschriebenen Ausführungsformen der Erfindung lassen sich mit der Formazanbildung kuppeln. Bei manchen dieser Reaktionen ist diese Kupplung aus Gleichgewichtsgründen sogar bevorzugt. Im folgenden sind die weiteren Ausführungsformen beschrieben.

E. 1. ADP + Formyl-P $\xrightarrow{\text{Formiat-kinase}}$ Formiat + ATP

2. Formiat + NAD$^+$ $\xrightarrow{\text{F-DH.}}$ CO$_2$ + NADH + H$_2$ + H$^+$

F-DH = Formiat-dehydrogenase

Zulässige Schwankungsbreite

Für Puffer und Pufferstärke gelten die Angaben zu Ausführung D.

pH 6,0 bis 10,0

Formiatkinase 0,05 bis 50 U/ml

Formiat-DH 0,1 bis 100 U/ml

F. 1. ADP + PEP $\xrightarrow{\text{PK}}$ Pyruvat + ATP

2. Pyruvat $\xrightarrow{\text{Pyruvat-decarboxylase}}$ Acetaldehyd + CO$_2$

3. Acetaldehyd + NAD(P)$^+$ + H$_2$O $\xrightarrow{\text{Aldehyd-DH}}$ Acetat + NAD(P) H + H$^+$

Zulässige Schwankungsbreite

Art und Stärke des Puffers : hier gelten die Ausführungen zur Ausführungsform D ebenfalls

pH 4,5 bis 7,5

PK 0,05 bis 50 U/ml

Al-DH 0,05 bis 50 U/ml

Pyr-DC 0,05 bis 50 U/ml

Besonders bevorzugt wird nachstehende Ausführungsform des erfindungsgemäßen Verfahrens.

G. 1. ADP + PEP $\xrightarrow{\text{PK}}$ Pyruvat + ATP

2. Pyruvat + Glutamat $\xrightarrow{\text{GPT}}$ Alanin + α-KG

3. α-KG + γ-Aminobutyrat $\xrightarrow{\text{γ-Aminobutyrat-Transaminase}}$ Glutamat + Succinat-semialdehyd

4. Succinat-semialdehyd + NADP$^+$ + H$_2$O $\xrightarrow{\text{SS-Al-DH}}$ Succinat + NADPH + H$^+$

GPT = Glutamat-pyruvat-transaminase

α-KG = α-Ketoglutarat

SS-Al-DH = Succinatsemialdehyd-dehydrogenase

Zulässige Schwankungsbreite

Für Puffer und Pufferstärke gilt das zu Ausführungsform D angegebene.

pH 6,0 bis 10,5

PK, GPT, GAB-GT, SS-AL-DH je 0,05 bis 50 U/ml

Ebenfalls besonders bevorzugt wird schließlich auch nachstehende Ausführungsform des erfindungsgemäßen Verfahrens.

H. 1. ADP + UTP $\xrightarrow{\text{NDPK}}$ ATP + UDP

2. UDP + Saccharose $\xrightarrow{\text{Saccharose-Synt}}$ UDPG + Fructose

3. UDPG + 2 NAD + H$_2$O $\xrightarrow{\text{UDPG-DH}}$ UDP-Glucuronat + 2 NADH NDPK = Nucleosid-diphosphatkinase

Zulässige Schwankungsbreite

Für Art und Stärke des Puffers gelten die Angaben zu Ausführungsform D.

pH-Bereich 6 bis 10,5

UDPG-DH 0,01 bis 15 U/ml

NDPK 1 bis 50 U/ml

Saccharose-Synt. 0,05 bis 10 U/ml

Die Einzelheiten für die Durchführung dieser Ausführungsformen sind bei den Beispielen, die diese Ausführungsformen näher erläutern, angeführt.

Wie schon erwähnt, ist die Anwendung der Erfindung besonders vorteilhaft für Reaktionen, bei denen ADP aus ATP gebildet wird. In der folgenden Tabelle sind daher Beispiele von Enzymen und Substraten aufgeführt, bei denen das zu messende ADP aus ATP gebildet wird und die Erfindung angewandt werden kann. Wie in den Beispielen näher erläutert, können auch hier gekoppelte Reaktionen ausgeführt werden. Sowohl Enzym als auch aufgeführte Substrate sind jeweils erfindungsgemäß bestimmbar. Welches erfindungsgemäße Verfahren im einzelnen angewandt werden soll, ist von der nachzuweisenden Reaktion im wesentlichen unabhängig.

Tabelle 1

| Enzym | (EC Nr.) | Substrate |
|---|---|---|
| Hexokinase | 2.7.1.1 | ATP/Hexosen |
| Glucokinase | 2.7.1.2 | ATP/Glucose |
| Fructokinase | 2.7.1.4 | ATP/Fructose |
| Galactokinase | 2.7.1.6 | ATP/Galactose |
| Mannokinase | 2.7.1.7 | ATP/Mannose |
| Glucosamin-kinase | 2.7.1.8 | ATP/2-Amino-2-desoxy-D-glucose |
| Phosphoglucokinase | 2.7.1.10 | ATP/Glucose-1-P |
| Phosphofructokinase | 2.7.1.11 | ATP/Fructose-6-P |
| Glucono-kinase | 2.7.1.12 | ATP/D-Gluconat |
| Adenosin-kinase | 2.7.1.20 | ATP/Adenosin |
| NAD-Kinase | 2.7.1.23 | ATP/NAD |
| Glycerin-kinase | 2.7.1.30 | ATP/Glycerin |
| Glycerat-kinase | 2.7.1.31 | ATP/Glycerat |
| Cholin-kinase | 2.7.1.32 | ATP/Cholin |
| Pyruvat-kinase | 2.7.1.40 | ATP/Pyruvat |
| Glucurono-kinase | 2.7.1.43 | ATP/Glucuronat |
| Galacturono-kinase | 2.7.1.44 | ATP/Galacturonat |
| Arabino-kinase | 2.7.1.54 | ATP/Arabinose |
| Mannitol-kinase | 2.7.1.57 | ATP/Mannit |
| Inosin-kinase | 2.7.1.73 | ATP/Inosin |
| Acetat-kinase | 2.7.2.1 | ATP/Acetat |
| Carbamat-kinase | 2.7.2.2 | $ATP/NH_3/CO_2$ |
| Asparat-kinase | 2.7.2.4 | ATP/Asparat |
| Carbamoyl-phosphat-synthase | 2.7.2.5 | $2 \ ATP/NH_3/CO_2 H_2O$ |
| Formiat-kinase | 2.7.2.6 | ATP/Formiat |
| Carbamoyl-phosphat-synthase | 2.7.2.9 | $2 \ ATP/Glutamin/CO_2/H_2O$ |
| Guanidinoacetat-kinase | 2.7.3.1 | ATP/Guanidinacetat |
| Creatin-kinase | 2.7.3.2 | ATP/Creatin |
| Arginin-kinase | 2.7.3.3 | ATP/L-Arginin |
| Ammoniak-kinase | 2.7.3.8 | $ATP/NH_3$ |
| Polyphosphat-kinase | 2.7.4.1 | ATP/(Phosphat) n |
| Adenylat-kinase | 2.7.4.3 | ATP/AMP |
| Nucleosid-monophosphat-kinase | 2.7.4.4 | ATP/Nucleosid-monophosphat |
| Nucleosid-diphosphat-kinase | 2.7.4.5 | ATP/Nucleosid-diphosphat |
| Guanylat-kinase | 2.7.4.6 | ATP/GMP |
| Cytidylat-kinase | 2.7.4.14 | ATP/CMP |
| Succinyl-CoA-Synthetase | 6.2.1.5 | ATP/Succinat/CoA |
| Glutaryl-CoA-Synthetase | 6.2.1.6 | ATP/Glutarat/CoA |
| Malyl-CoA-Synthetase | 6.2.1.9 | ATP/Malat/CoA |
| Glutamin-Synthetase | 6.3.1.2 | $ATP/Glutamat/NH_3$ |
| Asparagin-Synthetase | 6.3.1.4 | $ATP/Aspartat/NH_3$ |
| γ-Glutamyl-Cystein-Synthetase | 6.3.2.2 | ATP/Glutamat/Cystein |
| Glutathion-Synthetase | 6.3.2.3 | ATP/γ-Glutamyl-cystein/Glycin |
| D-Alanylalanin-Synthetase | 6.3.2.4 | ATP/2 D-Alanin |
| Harnstoff-carboxylase | 6.3.4.6 | $ATP/Harnstoff/CO_2$ |
| Pyruvat-carboxylase | 6.4.1.1 | $ATP/Pyruvat/H_2O/CO_2$ |
| 5'-Nucleosidase | 3.1.3.5 | $ATP/ASN/NH_3/Glutamat$ |
| Adenosin-Desaminase | 3.5.4.4 | $ATP/ASN/ISN/NH_3/Glutamat$ |

Ein weiterer Gegenstand der Erfindung ist ein Reagens zur Bestimmung von ADP mit einem Gehalt an einer Kinase, einem phosphorylierten, von ADP verschiedenen Substrat derselben und Puffer, welches dadurch gekennzeichnet ist, daß es eine für die entphosphorylierte Form dieses Substrats oder für ein transaminiertes, decarboxyliertes oder hexosidiertes Folgeprodukt derselben spezifische Dehydrogenase und das Coenzym der Dehydrogenase enthält. Zusätzlich kann dieses Reagens ein Tetrazolium-salz und einen dafür geeigneten Elektronenüberträger sowie gegebenenfalls ein oberflächenaktives Mittel oder/-und ein System zur Bildung von ADP aus ATP sowie die von den jeweils verwendeten Enzymen benötigten Salze enthalten. Das System zur Bildung von ADP aus ATP besteht entweder aus ATP und der für die jeweils unter ADP-Bildung zu bestimmenden substanzspezifischen Kinase odern falls es zur Aktivitätsbestimmung dieser Kinase verwendet werden soll, aus ATP und dem zweiten Substrat der Kinase.

**0 009 076**

Das erfindungsgemäße Reagens kann in trockener lyophilisierter Form oder als gebrauchsfertige wässrige Lösung vorliegen. In einer besonderen Ausführungsform kann es auch auf einem geeigneten Träger imprägniert oder inkorporiert als Teststreifen vorliegen.

In den nachfolgenden Beispielen werden folgende Abkürzungen verwendet:

| | |
|---|---|
| Al-DH | Aldehyd-dehydrogenase |
| Formiat-DH | Formiat-dehydrogenase |
| GPT | Glutamat-pyruvat-transaminase |
| GAB-GT | $\gamma$-Aminobutyrat-$\alpha$-ketoglutarat-transaminase |
| SS-Al-DH | Succinatsemialdehyd-dehydrogenase |
| GOT | Glutamat-oxalacetat-transaminase |
| NDPK | Nucleosid-5'-diphosphat-kinase |
| UTP | Uridin-5'-triphosphat |
| ADP | Adenosin-5'-diphosphat |
| ATP | Adenosin-5'-triphosphat |
| PEP | Phosphoenol-pyruvat |
| PK | Pyruvat-kinase |
| NAD | Nicotinamid-adenin-dinucleotid |
| NADH | Nicotinamid-adenin-dinucleotid, reduziert |
| NADP | Nicotinamid-adenin-dinucleotid-phosphat |
| NADPH | Nicotinamid-adenin-dinucleotid-phosphat, reduziert |
| CK | Creatin-kinase |
| MTT | 3-(4,5-Dimethylthiazolyl-2)-2,5-diphenyl-tetrazoliumbromid |
| Gal-1-P | D-Galactose-1-phosphat |
| Gal-Kinase | Galactose-kinase |
| Gal-DH | Galactose-dehydrogenase |
| UDPG-DH | Uridin-5'-diphospho-glucose-dehydrogenase |
| Pyr-DC | Pyruvat-decarboxylase |
| ADA | Adenosin-desamidase |

Die folgenden Beispiele erläutern die Erfindung weiter.

Beispiel 1

Bestimmung von ADP mit Gal-Kinase unter NADH-Bildung Temperatur 25 °C, Meßwellenlänge 365 nm, 1 cm Küvetten, Testvolumen 2,02 ml

| Ausgangslösung | Probe (ml) | Leerwert (ml) | Konzentration im Test |
|---|---|---|---|
| K-Phosphat-puffer, pH 7,5 0,1 Mol/l | 0,2 | 0,2 | 10 mMol/l |
| MgCl$_2$ 0,1 Mol/l | 0,2 | 0,2 | 10 mMol/l |
| Gal-1-P 60 mMol/l | 0,1 | 0,1 | 3 mMol/l |
| NAD 40 mMol/L | 0,1 | 0,1 | 2 mMol/l |
| Gal-Kinase 100 U/ml | 0,1 | 0,1 | 5 U/ml |
| Gal-DH 12,5 U/ml | 0,1 | 0,1 | 0,625 U/ml |
| H$_2$O | 1,2 | 1,2 | — |
| mischen, dann starten mit ADP-Lösung | 0,02 | — | — |

mischen, inkubieren, nach Stillstand der Extinktionszunahme Extinktion der Probe gegen Extinktion des Leerwerts messen. Aus der Differenz ergibt sich der ADP-Gehalt der Probe nach der Formel

$$ADP \text{ (mMol/l)} = \Delta E . 29,71$$

Beispiel 2

Bestimmung von ADP mit PK/Pyruvat-DC unter NADH-Bildung. Temperatur 25 °C, Meßwellenlänge 365 nm, Testvolumen 2 ml, 1 cm Küvetten

6

| Ausgangslösung | Probe (ml) | Leerwert (ml) | Konzentration im Test |
|---|---|---|---|
| Imidazol-puffer, 0,2 Mol/l   pH 6,5 | 1,0 | 1,0 | 0,1 mMol/l |
| $MgSO_4$   0,2 Mol/l | 0,1 | 0,1 | 10   mMol/l |
| NAD   40 mMol/l | 0,1 | 0,1 | 2   mMol/l |
| PEP   10 mMol/l | 0,1 | 0,1 | 0,5 mMol/l |
| Pyr-DC/PK/Al-DH je   100 U/ml | 0,05 | 0,05 | je 2,5 U/ml |
| $H_2O$ | 0,65 | 0,65 | — |
| mischen, dann starten mit ADP | 0,2 | — | — |

mischen, inkubieren, nach Stillstand der Extinktionszunahme Extinktion der Probe gegen Extinktion des Leerwerts bestimmen, Gehalt an ADP berechnen nach

$$ADP\ (mMol/l) = \Delta E \cdot 29,71$$

Beispiel 3

Bestimmung von ADP mit Formiat-kinase unter NADH-Bildung. Meßtemperatur 25 °C, Meßwellenlänge 365 nm, 1 cm Kübetten, Testvolumen 2,02 ml

| Konzentration der Ausgangslösung | Probe (ml) | Leerwert (ml) | Konzentration im Test |
|---|---|---|---|
| Dimethylglutarat pH 6,9   0,1 Mol/l | 1,0 | 1,0 | 50 mMol/l |
| Formylphosphat   0,1 Mol/l | 0,1 | 0,1 | 5 mMol/l |
| Formiatkinase   20 U/ml | 0,1 | 0,1 | 1 U/ml |
| Formiat-DH   200 U/ml | 0,1 | 0,1 | 10 U/ml |
| NAD   0,08 Mol/l | 0,1 | 0,1 | 4 mMol/l |
| $MnSO_4$   0,1 Mol/l | 0,1 | 0,1 | 5 mMol/l |
| $H_2O$ | 0,5 | 0,5 | — |
| mischen, dann starten mit ADP | 0,02 | — | — |

mischen, inkubieren, nach Stillstand der Extinktionszunahme Extinktion der Probe gegen Leerwert bestimmen, Gehalt an ADP berechnen nach

$$ADP\ (mMol/l) = \Delta E \cdot 29,71$$

Beispiel 4

Bestimmung von ADP mit GPT, GAB-GT und SS-Al-DH unter NADPH-Bildung, Meßtemperatur 25 °C, Meßwellenlänge 365 nm, 1 cm Küvetten, 2,22 ml Testvolumen

| Konzentration der Ausgangslösung | Probe (ml) | Leerwert (ml) | Konzentration im Test |
|---|---|---|---|
| Imidazol-puffer, pH 7,6   0,1 Mol/l | 1,0 | 1,0 | 50 mMol/l |
| $MgSO_4$   100 mMol/l | 0,1 | 0,1 | 5 mMol/l |
| PEP   20 mMol/l | 0,1 | 0,1 | 1 mMol/l |
| NADP   20 mMol/l | 0,1 | 0,1 | 1 mMol/l |
| Glutamat   0,6 Mol/l | 0,2 | 0,2 | 60 mMol/l |
| $\gamma$-Aminobutyrat   0,9 Mol/l | 0,2 | 0,2 | 90 mMol/l |
| PK   40 U/ml | 0,1 | 0,1 | 2   U/ml |
| GPT   100 U/ml | 0,1 | 0,1 | 5   U/ml |
| GAB-GT   50 U/ml | 0,1 | 0,1 | 2,5 U/ml |
| SS-Al-DH   50 U/ml | 0,1 | 0,1 | 2,5 U/ml |
| mischen, inkubieren, starten mit ADP | 0,02 | — | — |

mischen, Stillstand der Extinktionszunahme abwarten, aus der Differenz der Extinktion zwischen Probe und Leerwert den Gehalt an ADP berechnen.

$$ADP\ (mMol/l) = \Delta E . 30,29$$

Beispiel 5

Bestimmung von Creatinin unter Formazanbildung. Meßtemperatur 25 °C, Meßwellenlänge 576 nm, 1 cm Küvetten, 2,5 ml Testvolumen

| Konzentration der Ausgangslösung | Probe (ml) | Leerwert (ml) | Konzentration im Test |
|---|---|---|---|
| Glycin-puffer, enth. 5 % | 1,0 | 1,0 | 2 % |
| Triton X100, 0,25 Mol/l   pH 8,0 | | | 0,1 Mol/l |
| NADP   0,1 Mol/l | 0,05 | 0,05 | 2 mMol/l |
| ATP   0,05 Mol/l | 0,05 | 0,05 | 1 mMol/l |
| PEP   20 mMol/l | 0,05 . | 0,05 | 0,4 mMol/l |
| Glutamat   50 mMol/l | 0,05 | 0,05 | 1 mMol/l |
| γ-Aminobutyrat   0,475 Mol/l | 0,5 | 0,5 | 95 mMol/l |
| MgCl$_2$   0,1 Mol/l | 0,05 | 0,05 | 2 mMol/l |
| MTT   1,5 mMol/l | 0,5 | 0,5 | 0,3 mMol/l |
| CK   500 U/ml | | | 10 U/ml |
| PK   200 U/ml | | | 4 U/ml |
| GPT   200 U/ml | | | 4 U/ml |
| Diaphorase   25 U/ml | 0,05 | 0,05 | 0,5 U/ml |
| GAB-GT   200 U/ml | | | 4 U/ml |
| SS-Al-DH   200 U/ml | 0,05 | 0,05 | 4 U/ml |
| Probe | 0,05 | — | — |
| mischen, starten mit | | | |
| Creatininase   500 U/ml | 0,05 | 0,05 | 10 U/ml |

mischen, 15 Minuten inkubieren, nach Ende der Extinktionszunahme Extinktion der Probe gegen Leerwert messen, Creatiningehalt berechnen nach

$$Creatinin\ (mMol/l) = \Delta E . 2,994$$

Die Reaktionsbedingungen sind durch die optimierte Creatininbestimmung festgelegt. Für die erfindungsgemäße Anwendung der ADP-Bestimmung gilt das unter Beispiel 4 Gesagte.

Beispiel 6

Bestimmung der CK unter NADPH-Bildung.
Meßtemperatur 25 °C, Meßwellenlänge 365 nm, 1 cm Küvetten, 2,2 ml Testvolumen

| Konzentration der Ausgangslösung | Probe (ml) | Konzentration im Test |
|---|---|---|
| Glycin-puffer, pH 9   169 mMol/l | | 84,5 mMol/l |
| Creatin   64 mMol/l | | 32   mMol/l |
| NaCl | 1,1 | 76,5 mMol/l |
| MgCl$_2$   72,6 mMol/l | 0,1 | 3,3 mMol/l |
| ATP   35,2 mMol/l | 0,05 | 0,8 mMol/l |
| PEP   30,8 mMol/l | 0,05 | 0,7 mMol/l |
| NADP   44 mMol/l | 0,1 | 2   mMol/l |
| Glutamat   44 mMol/l | 0,05 | 1   mMol/l |
| γ-Aminobutyrat   0,418 Mol/l | 0,5 | 95   mMol/l |
| PK   176 U/ml | | 4 U/ml |
| GPT   176 U/ml | | 4 U/ml |
| GAB-GT   176 U/ml | | 4 U/ml |
| SS-Al-DH   176 U/ml | 0,05 | 4 U/ml |
| Probe | 0,2 | — |

mischen, 2 Minuten inkubieren, dann nach je 1 Minute $\Delta E$ ablesen und die CK-Menge in der Probe berechnen nach

$$CK \; (U/ml) = \Delta E/min \, . \, 3{,}143$$

Die Reaktionsbedingungen sind durch die optimierte Creatinkinase-Bestimmung festgelegt. Für die erfindungsgemäße Anwendung der ADP-Bestimmung gilt das unter Beisp. 4 Gesagte.

## Beispiel 7

Bestimmung von ADP unter NADH-Bildung.
Meßtemperatur 25 °C, Meßwellenlänge 365 nm, 1 cm Küvetten, Testvolumen 1,6 ml

| Ausgangslösung | ml | Endkonzentration im Test |
|---|---|---|
| Glycylglycin, pH 8,0   0,05 mMol/l | 2,1 | 33,0  mMol/l |
| NAD   35 mMol/l | 0,1 | 1,13 mMol/l |
| Saccharose   500 mMol/l | 0,5 | 78,1  mMol/l |
| UDPG-DH   3 U/ml | 0,05 | 0,05 U/ml |
| NDPK   400 U/ml | 0,05 | 6,25 U/ml |
| UTP   16,5 mMol/l | 0,1 | 0,52 mMol/l |
| Saccharose-synthetase   12 U/ml | 0,1 | 0,38 U/ml |

mischen, pipettieren in Mikrokübetten

| | Leerwert (ml) | Probe (ml) |
|---|---|---|
| Mix | 1,5 | 1,5 |
| $H_2O$ | 0,1 | — |
| Probe | — | 0,1 |

mischen, nach Stillstand der Extinktionszunahme Extinktion der Probe gegen Leerwert messen und ADP-Gehalt berechnen nach

$$ADP \; (mMol/l) = \Delta E \, . \, 2{,}353$$

Zulässige Schwankungsbreite :
Art und Stärke des Puffers :
Siehe Beispiel 1
pH-Bereich 6,0-10,5
UDPG-DH 0,01-15 U/ml
NDPK 0,2-50 U/ml
Sacchar. Synthetase 0,05-10 U/ml

## Beispiel 8

Bestimmung von Creatinin unter NADH-Bildung
Meßtemperatur 25 °C, Meßwellenlänge 365 nm, 1 cm Mikroküvetten, Testvolumen 1,55 ml.
Herstellung des Reagentienmixes

| Konzentration der Ausgangslösung | ml | Konzentration im Test |
|---|---|---|
| Glycylglycin, pH 8,0   0,1 mMol/l | 1,9 | 61    mMol/l |
| NAD   35 mMol/l | 0,1 | 1,13 mMol/l |
| Saccharose   0,5 Mol/l | 0,5 | 80,6  mMol/l |
| UDPG-DH   3 U/ml | 0,05 | 0,05 U/ml |
| NDPK   400 U/ml | 0,05 | 6,45 U/ml |
| UTP   16,5 mMol/l | 0,1 | 0,53 mMol/l |
| ATP   16,7 mMol/l | 0,1 | 0,54 mMol/l |
| Saccharose Synth.   12 U/ml | 0,1 | 0,38 U/ml |
| CK   500 U/ml | 0,05 | 8    U/ml |
| Probe | 0,05 | — |

mischen, in Mikroküvette pipettieren.

| | Leerwert (ml) | Probe (ml) |
|---|---|---|
| Mix | 1,5 | 1,5 |
| H₂O | 0,05 | — |
| Creatininase, 350 U/ml | — | 0,05 |

Nach Stillstand der Extinktionszunahme Extinktion der Probe gegen Extinktion des Testleervolumens messen und Creatinin-gehalt der Probe berechnen nach

$$Creatinin\ (mMol/l) = \Delta E \cdot 9{,}12$$

Die Reaktionsbedingungen sind durch die optimierte Creatininbestimmung festgelegt. Für die erfindungsgemäße Anwendung der ADP-Bestimmung gilt das unter Beispiel 7 Gesagte.

Beispiel 9

Bestimmung von Ammoniak unter NADH-Bildung.
Meßtemperatur 25 °C, Meßwellenlänge 365 nm, 1 cm Mikroküvetten, Testvolumen, 1,6 ml.
Die verwendeten Enzyme sollten weitgehend frei von Ammoniumionen sein.
Herstellung des Reagentienmixes :

| Konzentration der Ausgangslösung | ml | Endkonzentration im Test |
|---|---|---|
| Imidazol-puffer, pH 7,8   0,1 Mol/l | 1,5 | 47    mMol/l |
| Glutaminsäure   0,1 Mol/l | 0,2 | 6,2  mMol/l |
| MgCl₂   0,41 Mol/l | 0,1 | 12,8  mMol/l |
| KCl   0,26 Mol/l | 0,1 | 8,1  mMol/l |
| NAD   35 mMol/l | 0,1 | 1,09 mMol/l |
| Saccharose   0,5 Mol/l | 0,5 | 78,1  mMol/l |
| UTP   16,5 mMol/l | 0,1 | 0,52 mMol/l |
| ATP   32,3 mMol/l | 0,1 | 1,01 mMol/l |
| Glutamin-synthetase   3 U/ml | 0,05 | 0,05 U/ml |
| Saccharose-synthetase   12 U/ml | 0,1 | 0,38 U/ml |
| NDPK   400 U/ml | 0,05 | 6,25 U/ml |
| UDPG-DH   3 U/ml | 0,1 | 0,09 U/ml |

mischen, in Mikroküvetten pipettieren.

| | Leerwert (ml) | Probe (ml) |
|---|---|---|
| Mix | 1,5 | 1,5 |
| H₂O | 0,1 | — |
| Probe | — | 0,1 |

Nach Stillstand der Extinktionszunahme Extinktion der Probe gegen Extinktion des Leerwerts

10

**0 009 076**

messen und Ammoniak-gehalt der Probe berechnen nach

$$\text{Ammoniak (mMol/l)} = \Delta E \cdot 2{,}253$$

Zulässige Schwankungsbreite :
Art und Stärke des Puffers : siehe Beispiel 1
pH-Bereich 6,9 bis 7,8
UDPG-DH 0,02 bis 15 U/ml
Saccharose-synthetase 0,1 bis 10 U/ml
NDPK 0,2 bis 50 U/ml
Glutamin-synthetase 0,02 bis 10 U/ml

Beispiel 10

Bestimmung von Ammoniak unter NADH-Bildung.
Die Bestimmung kann auch mit Hilfe der Carbamat-kinase (z.B. aus Streptococcus faecalis) ausgeführt werden.
Meßtemperatur 37 °C, Meßwellenlänge 365 nm, 1 cm Mikroküvetten Testvolumen 1,6 ml.
Herstellung eines Reagentienmixes :

| Konzentration der Ausgangslösung | ml | Konzentration im Test |
|---|---|---|
| Imidazol-puffer, pH 7,8   0,1 Mol/l | 1,5 | 47    mMol/l |
| Kaliumcarbonat  0,5 Mol/l | 0,2 | 31,2 mMol/l |
| MgCl$_2$  0,41 Mol/l | 0,1 | 12,8 . mMol/l |
| NAD  35 mMol/l | 0,1 | 1,09 mMol/l |
| Saccharose  0,5 Mol/l | 0,5 | 78,1  mMol/l |
| UTP  15,6 mMol/l | 0,1 | 0,52 mMol/l |
| ATP  32,3 mMol/l | 0,1 | 1,01 mMol/l |
| UDPG-DH  3 U/ml | 0,1 | 0,09 U/ml |
| NDPK  400 U/ml | 0,05 | 6,25 U/ml |
| Saccharose-synthetase  12 U/ml | 0,1 | 0,38 U/ml |
| Carbamat-kinase  100 U/ml | 0,15 | 4,69 U/ml |

mischen, in Mikroküvetten pipettieren.

| | Leerwert (ml) | Probe (ml) |
|---|---|---|
| Mix | 1,5 | 1,5 |
| H$_2$O | 0,1 | — |
| Probe | —. | 0,1 |

Bei 37 °C etwa 20 Minuten stehenlassen.
Nach Ende der Extinktionszunahme Extinktion der Probe gegen Extinktion des Leerwerts messen.
Ammoniak-gehalt berechnen nach :

$$\text{Ammoniak (mMol/l)} = \Delta E \cdot 4{,}706$$

Beispiel 11

Bestimmung von NH$_3$ unter NADPH-Bildung
Meßtemperatur 25 °C, Meßwellenlänge 365 nm, 1 cm-Küvetten, Testvolumen 2,25 ml.

11

| Konzentration der Ausgangslösung | Probe (ml) | Leerwert (ml) | Konzentration im Test |
|---|---|---|---|
| Imidazol-puffer, pH 7,0   500 mMol/l | 1,0 | 1,0 | 222   mMol/l |
| KCl   400 mMol/l | 0,1 | 0,1 | 18   mMol/l |
| $MgCl_2$   800 mMol/l | 0,1 | 0,1 | 35   mMol/l |
| NADP   20 mMol/l | 0,1 | 0,1 | 0,9 mMol/l |
| ATP   32 mMol/l | 0,1 | 0,1 | 1,4 mMol/l |
| PEP   20 mMol/l | 0,1 | 0,1 | 0,9 mMol/l |
| Glutamat   100 mMol/l | 0,2 | 0,2 | 8,9 mMol/l |
| γ-Aminobutyrat   900 mMol/l | 0,2 | 0,2 | 80   mMol/l |
| PK   30 U/ml |  |  | 1,3 U/ml |
| GPT   60 U/ml | 0,1 | 0,1 | 2,7 U/ml |
| GAB-GT   60 U/ml |  |  | 2,7 U/ml |
| SS-DH   50 U/ml |  |  | 2,2 U/ml |
| Probe | 0,2 | — | — |
| Wasser | — | 0,2 | — |

mischen, Vorrekation ablaufen lassen,starten mit

| Glutamin-Synthetase   10 U/ml | 0,05 | 0,05 | 0,2 U/ml |

mischen, ca. 20 min inkubieren, nach Ende der Extinktionszunahme Extinktion der Probe gegen Leerwert bestimmen, Gehalt an $NH_3$ berechnen nach

$$NH_3 \ (mMol/l) = \Delta E . 3,214$$

Zulässige Schwankungsbreite :
Art und Stärke des Puffers : Phosphat-, Succinat-, Imidazol-, bedingt auch TRIS- und TRA-Puffer pH 6,8 bis 7,8.
PK, GPT, GAB-GT, SS-DH : je 0,05-50 U/ml
Glutamin-Synthetase : 0,02-10 U/ml

Beispiel 12

Bestimmung von 5'-Nucleotidase unter NADPH-Bildung.
Meßtemperatur 25 °C, Meßwellenlänge 365 nm, 1 cm-Küvetten, Testvolumen 2,25 ml.

| Konzentration der Ausgangslösung | Probe (ml) | Leerwert (ml) | Konzentration im Test |
|---|---|---|---|
| K-Phosphat-puffer, pH 7,2   100 mMol/l | 1,0 | 1,0 | 444   mMol/l |
| KCl   400 mMol/l | 0,1 | 0,1 | 18   mMol/l |
| $MgCl_2$   800 mMol/l | 0,1 | 0,1 | 35   mMol/l |
| NADP   20 mMol/l | 0,1 | 0,1 | 0,9 mMol/l |
| ATP   32 mMol/l | 0,1 | 0,1 | 1,4 mMol/l |
| PEP   20 mMol/l | 0,1 | 0,1 | 0,9 mMol/l |
| Glutamat   100 mMol/l | 0,2 | 0,2 | 8,9 mMol/l |
| γ-Aminobutyrat   900 mMol/l | 0,2 | 0,2 | 80   mMol/l |
| β-Glycerophosphat   80 mMol/l | 0,1 | 0,1 | 3,5 mMol/l |
| PK   30 U/ml |  |  | 1,3 U/ml |
| GPT   60 U/ml |  |  | 2,7 U/ml |
| GAB-GT   60 U/ml | 0,1 | 0,1 | 2,7 U/ml |
| SS-DH   50 U/ml |  |  | 2,2 U/ml |
| ADA   200 U/ml |  |  | 8,8 U/ml |
| Probe | 0,1 | 0,1 |  |

mischen, ca. 10 min inkubieren, dannstarten mit

| AMP   80 mMol/l | 0,05 | — | 1,8 mMol/l |

5'-Nucleotidase (U/ml) = $\Delta E$/min. 6,43

**0 009 076**

Zulässige Schwankungsbreite :
Puffer : Phosphat, Succinat
pH : 6,8-7,3
ADA : 1,2-20 U/ml

**Ansprüche**

1. Verfahren zur quantitativen enzymatischen Bestimmung von Adenosindiphosphat (ADP) in wässriger Lösung durch Phosphorylierung mit einer Kinase in Gegenwart eines phosphorylierten Substrats derselben unter Bildung von ATP und entphosphoryliertem Substrat, dadurch gekennzeichnet, daß das entphosphorylierte Substrat, gegebenenfalls unter Zwischenschaltung einer weiteren Hilfsreaktion mit NAD(P) in Gegenwart einer Dehydrogenase umgesetzt und gebildetes NAD(P)H direkt gemessen oder mit einem Tetrazoliumsalz in Gegenwart eines Elektronenüberträgers umgesetzt wird unter Bildung eines Formazans und letzteres gemessen wird.

2. Verfahren nach Anspruch 1, dadurch gekennzeichnet, daß als entphosphoryliertes Substrat Galactose, Formiat, Pyruvat oder Nucleosid-diphosphat verwendet wird.

3. Verfahren nach Anspruch 1 oder 2, dadurch gekennzeichnet, daß als zwischengeschaltete Hilfsreaktion eine Decarboxylierung, Transaminierung oder Hexosidierung verwendet wird.

4. Verfahren nach einem der vorhergehenden Ansprüche, dadurch gekennzeichnet, daß man

a) ADP mit Galacto-kinase und Galactose-1-Phosphat umsetzt und mit der gebildeten Galactose NAD in Gegenwart von Galactose-dehydrogenase reduziert oder

b) ADP mit Formiat-kinase und Formylphosphat umsetzt und mit dem gebildeten Formiat in Gegenwart von Formiat-dehydrogenase NAD reduziert, oder

c) ADP mit Pyruvat-kinase und Phosphoenol-pyruvat umsetzt, gebildetes Pyruvat mit Pyruvatdecarboxylase decarboxyliert und mit dem gebildeten Acetaldehyd und Aldehyddehydrogenase NAD reduziert oder

d) nach c) gebildetes Pyruvat mit Glutamat und GPT (Glutamat-pyruvat-transaminase) in Alanin und $\alpha$-Ketoglutarat transaminiert, letzteres mit $\gamma$-Aminobutyrat und $\gamma$-Aminobutyrat-transaminase in Glutamat und Succinat-semialdehyd überführt und letzteres mit Succinat-semialdehyd-dehydrogenase zur Reduktion von NADP heranzieht oder

e) ADP mit UTP und Nucleosid-diphosphat-kinase umsetzt, gebildetes UDP mit Saccharose und Saccharose-synthetase in Fructose und Uridindiphosphat-glucose umwandelt und mit letzterer und Uridindiphosphat-glucosedehydrogenase NAD reduziert.

5. Reagens zur Bestimmung von ADP mit einem Gehalt an einer Kinase, einem phosphorylierten, von ADP verschiedenen Substrat derselben und Puffer, dadurch gekennzeichnet, daß es eine für die entphosphorylierte Form des Substrats oder für ein transaminiertes, decarboxyliertes oder hexosidiertes Folgeprodukt derselben spezifische Dehydrogenase und das Coenzym der Dehydrogenase enthält.

6. Reagens nach Anspruch 5, dadurch gekennzeichnet, daß es zusätzlich ein Tetrazoliumsalz, einen dafür geeigneten Elektronenüberträger und gegebenenfalls ein oberflächenaktives Mittel enthält.

7. Reagens nach Anspruch 5 oder 6, dadurch gekennzeichnet, daß es Galactose-kinase, Galactose-1-phosphat, Galactose-dehydrogenase und NAD enthält.

8. Reagens nach Anspruch 7, dadurch gekennzeichnet, daß es 0,1 bis 20 U/ml Galactose-kinase, 0,05 bis 20 U/ml Galactose-dehydrogenase, bezogen auf fertige wässrige Lösung, und Puffer, pH 6,0 bis 10,5, enthält.

9. Reagens nach Anspruch 5 oder 6, dadurch gekennzeichnet, daß es Formiat-kinase, Formylphosphat, Formyldehydrogenase und NAD enthält.

10. Reagens nach Anspruch 9, dadurch gekennzeichnet, daß es 0,05 bis 50 U/ml Formiat-kinase, 0,1 bis 100 U/ml Formiat-dehydrogenase, bezogen auf fertige wässrige Lösung, und Puffer, pH 6,0 bis 10,0 enthält.

11. Reagens nach Anspruch 5 oder 6, dadurch gekennzeichnet, daß es Pyruvat-kinase, Phosphoenol-pyruvat, Glutamat, Glutamat-pyruvat-transaminase, $\gamma$-Aminobutyrat, $\gamma$-Aminobutyrat-transaminase, Succinat-semialdehyd-dehydrogenase und NADP enthält.

12. Reagens nach Anspruch 11, dadurch gekennzeichnet, daß es die Enzyme in einer Menge von je 0,05 bis 50 U/ml, bezogen auf fertige wässrige Lösung, und Puffer, pH 6,0 bis 10,5 enthält.

13. Reagens nach Anspruch 5 oder 6, dadurch gekennzeichnet, daß es Nucleosid-diphosphat-kinase, Uridin-triphosphat, Saccharose, Saccharose-synthetase, Uridin-diphosphat-glucose-dehydrogenase und NAD enthält.

14. Reagens nach Anspruch 13, dadurch gekennzeichnet, daß es 0,01 bis 15 U/ml Uridin-diphosphat-glucose-dehydrogenase, 1 bis 50 U/ml Nucleosid-diphosphat-kinase, 0,05 bis 10 U/ml Saccharose-synthetase und Puffer, pH 6,0 bis 10,5 enthält.

15. Reagens nach einem der Ansprüche 5 bis 14, dadurch gekennzeichnet, daß es zusätlich ein System zur Bildung von ADP aus ATP enthält.

16. Reagens nach einem der Ansprüche 6 bis 15, dadurch gekennzeichnet, daß es auf einem Teststreifen imprägniert oder inkorporiert vorliegt.

13

# 0 009 076

## Claims

1. Process for the quantitative enzymatic determination of adenosine diphosphate (ADP) in aqueous solution by phosphorylation with a kinase in the presence of a phosphorylated substrate thereof, with the formation of ATP and dephosphorylated substrate, characterised in that the dephosphorylated substrate, possibly with the interpolation of a further adjuvant reaction, is reacted with NAD(P) in the presence of a dehydrogenase and the NAD(P)H formed is measured directly or is reacted with a tetrazolium salt in the presence of an electron carrier, with the formation of a formazane and the latter is measured.

2. Process according to claim 1, characterised in that the galactose, formate, pyruvate or nucleoside-diphosphate is used as dephosphorylated substrate.

3. Process according to claim 1 or 2, characterised in that a decarboxylation, transamination or hexosidation is used as the interpolated adjuvant reaction.

4. Process according to one of the preceding claims, characterised in that one
a) reacts ADP with galactokinase and galactose-1-phosphate and reduces NAD with the galactose formed in the presence of galactose dehydrogenase or
b) reacts ADP with formate kinase and formyl phosphate and reduces NAD with the formate formed in the presence of formate dehydrogenase or
c) reacts ADP with pyruvate kinase and phosphoenol-pyruvate, decarboxylates pyruvate formed with pyruvate decarboxylase and reduces NAD with the acetaldehyde formed and aldehyde dehydrogenase or
d) transaminates pyruvate formed according to c) with glutamate and GPT (glutamate-pyruvate transaminase) into alanine and $\alpha$-ketoglutarate, converts the latter with $\gamma$-aminobutyrate and $\gamma$-aminobutyrate transaminase into glutamate and succinate-semialdehyde and uses the latter, with succinate-semialdehyde dehydrogenase, for the redaction of NADP or
e) reacts ADP with UTP and nucleoside-diphosphate kinase, converts UDP formed with saccharose and saccharose synthetase into fructose and uridine-diphosphate-glucose and reduces NAD with the latter and uridine-diphosphate-glucose dehydrogenase.

5. Reagent for the determination of ADP with a content of a kinase, of a phosphorylated substrate thereof different from ADP and buffer, characterised in that it contains a dehydrogenase specific for the dephosphorylated form of the substrate or for a transaminated, decarboxylated or hexosidised subsequent product thereof and the co-enzyme of the dehydrogenase.

6. Reagent according to claim 5, characterised in that it additionally contains a tetrazolium salt, an electron carrier suitable therefor and possibly a surface-active agent.

7. Reagent according to claim 5 or 6, characterised in that it contains galactose kinase, galactose-1-phosphate, galactose dehydrogenase and NAD.

8. Reagent according to claim 7, characterised in that it contains 0.1 to 20 U/ml. galactose kinase, 0.05 to 20 U/ml. galactose dehydrogenase, referred to the final aqueous solution, and buffer, pH 6.0 to 10.5.

9. Reagent according to claim 5 or 6, characterised in that it contains formate kinase, formyl phosphate, formyl dehydrogenase and NAD.

10. Reagent according to claim 9, characterised in that it contains 0.05 to 50 U/ml. formate kinase, 0.1 to 100 U/ml. formate dehydrogenase, referred to the final aqueous solution, and buffer, pH 6.0 to 10.0.

11. Reagent according to claim 5 or 6, characterised in that it contains pyruvate kinase, phosphoenol pyruvate, glutamate, glutamate-pyruvate transaminase, $\gamma$-aminobutyrate, $\gamma$-aminobutyrate transaminase, succinate-semialdehyde dehydrogenase and NADP.

12. Reagent according to claim 11, characterised in that it contains the enzymes in an amount of, in each case, 0.05 to 50 U/ml., referred to the final aqueous solution, and buffer, pH 6.0 to 10.5.

13. Reagent according to claim 5 or 6, characterised in that it contains nucleoside-diphosphate kinase, uridine triphosphate, saccharose, saccharose synthetase, uridine-diphosphate-glucose dehydrogenase and NAD.

14. Reagent according to claim 13, characterised in that it contains 0.01 to 15 U/ml. uridine-diphosphate-glucose dehydrogenase, 1 to 50 U/ml. nucleoside-diphosphate kinase, 0.05 to 10 U/ml. saccharose synthetase and buffer, pH 6.0 to 10.5.

15. Reagent according to one of claims 5 to 14, characterised in that it additionally contains a system for the formation of ADP from ATP.

16. Reagent according to one of claims 6 to 15, characterised in that it is present impregnated or incorporated on to a test strip.

## Revendications

1. Procédé pour la détermination enzymatique quantitative de l'acide adénosine-diphosphorique (ADP) en solution aqueuse par phosphorylation au moyen d'une kinase en présence d'un substrat phosphorylé de celle-ci avec formation d'ATP et de substrat déphosphorylé, caractérisé en ce que l'on fait réagir le substrat déphosphorylé, éventuellement avec intercalation d'une réaction auxiliaire supplémentaire, avec le NAD(P) en présence d'une déshydrogénase et on mesure directement le NAD(P)H formé ou on le fait réagir avec un sel de tétrazolium en présence d'un agent de transfert d'électrons avec formation d'une formazan et on mesure ce dernier.

14

**0 009 076**

2. Procédé selon la revendication 1, caractérisé en ce que l'on utilise, en tant que substrat déphosphorylé, du galactose, du formiate, du pyruvate ou du nucléoside-diphosphate.

3. Procédé selon la revendication 1 ou 2, caractérisé en ce que l'on utilise, en tant que réaction auxiliaire intercalaire, une décarboxylation, transamination ou hexosidation.

4. Procédé selon l'une des revendications précédentes, caractérisé en ce que

a) on fait réagir l'ADP avec de la galacto-kinase et du galactose-1-phosphate et on réduit, en présence de galactose-déshydrogénase, le NAD au moyen de la galactose formée, ou

b) on fait réagir l'ADP avec de la formiate-kinase et du formyl-phosphate et on réduit, en présence de formiate-déshydrogénase, le NAD au moyen du formiate formé, ou

c) on fait réagir l'ADP avec de la pyruvate-kinase et du phosphoénol-pyruvate, on décarboxyle le pyruvate formé au moyen de pyruvate-décarboxylase et on réduit le NAD au moyen de l'acétaldéhyde formé et d'aldéhyde-déshydrogénase ou

d) on transamine, au moyen de glutamate et de GPT (glutamate-pyruvate-transaminase), le pyruvate formé selon c) en alanine et α-cétoglutarate, on transforme ce dernier au moyen de γ-amino-butyrate et de γ-aminobutyrate-transaminase, en glutamate et succinate-semialdéhyde et on soumet ce dernier à oxydation par le NADP au moyen de succinate-semialdéhyde-déshydrogénase ou

e) on fait réagir l'ADP avec de l'UTP et de la nucléoside-diphosphate-kinase, on transforme, au moyen de saccharose et de saccharose-synthétase, l'UDP formé en fructose et uridine-diphosphate-glucose et on réduit le NAD au moyen de ce dernier et d'uridine-diphosphate-glucose-déshydrogénase.

5. Réactif, pour la détermination de l'ADP avec une teneur en une kinase, en un substrat phosphorylé de celle-ci, différent de l'ADP et en tampon, caractérisé en ce qu'il contient une déshydrogénase spécifique pour la forme déphosphorylée du substrat ou pour un produit transaminé, décarboxylé ou hexosidé résultant de celle-ci et le coenzyme de la déshydrogénase.

6. Réactif selon la revendication 5, caractérisé en ce qu'il contient en outre un sel de tétrazolium, un agent de transfert des électrons approprié à celui-ci et éventuellement un agent tensio actif.

7. Réactif selon la revendication 5 ou 6, caractérisé en ce qu'il contient de la galactose-kinase, du galactose-1-phosphate, de la galactose-déshydrogénase et du NAD.

8. Réactif selon la revendication 7, caractérisé en ce qu'il contient 0,1 à 20 U/ml de galactose-kinase, 0,05 à 20 U/ml de galactose-déshydrogénase, par rapport à la solution aqueuse terminée, et du tampon à pH 6,0 à 10,5.

9. Réactif selon la revendication 5 ou 6, caractérisé en ce qu'il contient de la formiate-kinase, du formylphosphate, de la formyl-déshydrogénase et du NAD.

10. Réactif selon la revendication 9, caractérisé en ce qu'il contient 0,05 à 50 U/ml de formiate-kinase, 0,1 à 100 U/ml de formiate-déshydrogénase, par rapport à la solution aqueuse terminée, et du tampon, à pH 6,0 à 10,0.

11. Réactif selon la revendication 5 ou 6, caractérisé en ce qu'il contient de la pyruvate-kinase, du phosphoénol-pyruvate, du glutamate, de la glutamate-pyruvate-transaminase, du γ-aminobutyrate, de la γ-aminobutyrate-transaminase, de la succinate-semialdéhyde-déshydrogénase et du NADP.

12. Réactif selon la revendication 11, caractérisé en ce qu'il contient les enzymes chacune en une quantité de 0,05 à 50 U/ml, par rapport à la solution aqueuse terminée, et du tampon, à pH 6,0 à 10,5.

13. Réactif selon la revendication 5 ou 6, caractérisé en ce qu'il contient de la nucléoside-diphosphate-kinase, de l'uridine-triphosphate, du saccharose, de la saccharose-synthétase, de l'uridine-diphosphate-glucose-déshydrogénase et du NAD.

14. Réactif selon la revendication 13, caractérisé en ce qu'il contient 0,01 à 15 U/ml d'uridine-diphosphate-glucose-déshydrogénase, 1 à 50 U/ml de nucléoside-diphosphate-kinase, 0,05 à 10 U/ml de saccharose-synthétase et du tampon, à pH 6,0 à 10,5.

15. Réactif selon l'une des revendications 5 à 14, caractérisé en ce qu'il contient en outre un système pour la formation d'ADP à partir d'ATP.

16. Réactif selon l'une des revendications 6 à 15, caractérisé en ce qu'il se présente imprégné ou incorporé sur une bande pour essais.

15